# EUROPEAN PATENT SPECIFICATION

(11) **EP 1 857 075 B1**
(45) Date of publication and mention of the grant of the patent: **09.04.2014**
(21) Application number: 07108350.5
(22) Date of filing: 16.05.2007
(51) Int. Cl.: A61F 2/16

(54) **Insertion device for intraocular lens**
Einsetzvorrichtung für Intraokularlinse
Dispositif d'insertion pour lentille intraoculaire

(30) Priority: 17.05.2006 JP 2006138225
(43) Date of publication of application: 21.11.2007
(73) Proprietor: STAAR Japan Inc., Urayasu-shi Chiba 279-0012 (JP)
(72) Inventor: Hishinuma, Yasuhiro, Ichikawa-shi Chiba (JP); Ohno, Kenichiro, Ichikawa-shi Chiba (JP); Shinzaki, Kenichi, Suzaka-shi Nagano (JP); Wakatsuki, Mitsuru, Ichikawa-shi Chiba (JP)
(74) Representative: TBK

(56) References cited:
- EP-A- 1 344 503
- EP-A1- 1 481 652
- US-A1- 2004 127 911

## Description

### BACKGROUND OF THE INVENTION

The present invention relates to an insertion device according to the preamble of claim 1 for inserting into an eye an intraocular lens which is inserted instead of a crystal lens after the crystal lens is extracted because of cataract and inserted into an eye in order to cure abnormal refraction.

In operations for cataract, an operation method for inserting an artificial intraocular lens into an eye through a small incision formed in an eyeball by using the flexibility of the lens and thereby folding and deforming the lens into a small shape is a mainstream. Then, in the case of an operation, an insertion device is frequently used which deforms a lens mounted to the main body of the device into a small shape while moving the lens in the main body of the device by a pushing shaft and pushes out the lens into an eye from a front end opening of an insertion cylinder inserted into an incision (see JP-A-2001-104347).

This insertion device is used not only for the operation of cataract but also for a lens inserting operation for an eyesight correction medical treatment.

In the insertion device disclosed in JP-A-2001-104347, an intraocular lens is held by a lens holding portion of the insertion device in a state in which a stress is not substantially applied to an optical portion of the lens, thereby allowing the lens to be housed in the insertion device and stored for a long time. In the case of an operation, a lens moving mechanism provided in the insertion device moves the lens to a position where the lens can be pushed out by the pushing shaft (an insertion preparing position) while being deformed to some extent, and then the pushing shaft is operated to deform the lens into a small shape and insert the lens into the eye.

The insertion device disclosed in JP-A- 2001-104347, however, requires two-step operations: an operation of the lens moving mechanism for moving the lens from the lens holding portion to the insertion preparing position, and an operation of the pushing shaft for pushing the lens into the eye. It is easier in operation and more convenient for an operator to be able to push the lens into the eye from a stored state only by the operation of the pushing shaft.

Document US-A1-2004/0127911 discloses a generic insertion device for an intraocular lens. The insertion device comprises a lens holding portion that holds the lens and a pushing shaft that moves the lens from the lens holding portion in a front end direction of the insertion device wherein the lens which is.moved by the pushing shaft is inserted into an eye. The lens holding portion includes a first holding portion which is allowed to contact a surface of a marginal portion of the lens on a first side of the lens and a second holding portion which is allowed to contact another surface of the marginal portion of the lens on a second side of the lens opposite to the first side when a position in the front end direction in the marginal portion with respect to the center of the lens is a front end position.

It is the object of the invention to provide an improved insertion device and system as well as a method for manufacturing the insertion system.

This object is achieved with an insertion device having the features of claim 1, an insertion system having the features of claim 2 and a method for manufacturing an insertion system having the features of claim 3. Advantageous further formations are subject of the dependent claims.

The present invention provides an insertion device for an intraocular lens which is capable of pushing out the lens into an eye by a pushing shaft directly from a state in which the lens is stored.

The present invention in its first aspect provides an insertion device for an intraocular lens as specified in claim 1.

The present invention in its second aspect provides an insertion system for an intraocular lens as specified in claim 2.

Further objects and features of the present invention will be become more apparent from the following description of preferred embodiments with reference to the drawings.

### BRIEF DESCRIPTION OF THE DRAWINGS

FIGS. 1A and 1B are a top view and a side sectional view, respectively, of an insertion device for an intraocular lens that is an embodiment of the present invention;
FIGS. 2A and 2B are a top view and a side sectional view, respectively, of the insertion device of the embodiment in a state of pushing out a lens;
FIG. 3A is a top view of the configuration of a lens support member in the insertion device of the embodiment;
FIG. 3B is a front sectional view of the configuration of the lens support member in the insertion device of the embodiment;
FIG. 3C is a side sectional view of the configuration of the lens support member in the Insertion device of the embodiment;
FIG. 4 is a sectional view, partially enlarged, of the lens support member of the embodiment;
FIGS. 5A and 5B are a bottom view and a side sectional view, respectively, of the configuration of a cover member in the insertion device of the embodiment;
FIGS. 6A and 6B are a top view and a side sectional view, respectively, of a state in which the cover member is assembled to the lens support member of the embodiment;
FIG. 7 is a top view of an insertion device of Comparative Example 1;
FIG. 8 is a top view of an insertion device of Comparative Example 2; and
FIGS. 9A and 9B are a top view and a side sectional view, respectively, of an insertion device of Comparative Example 3.

### DESCRIPTION OF THE EMBODIMENTS

Now, a preferred embodiment of the present invention will be described with reference to the accompanying drawings.

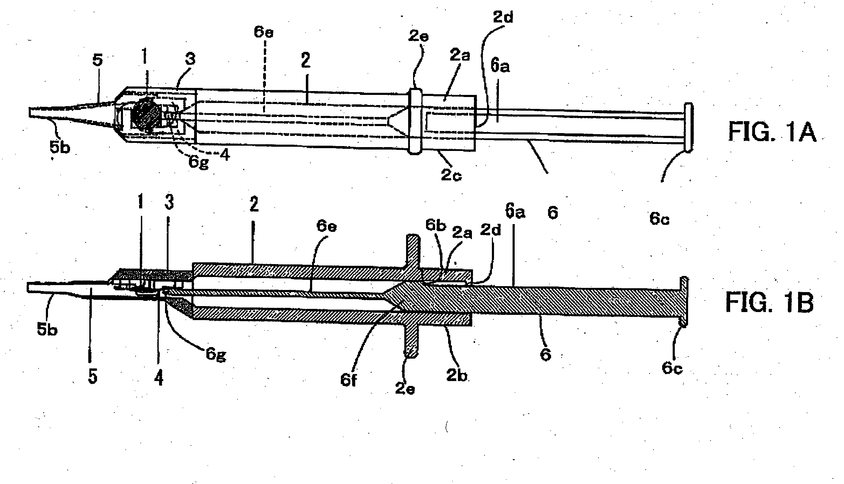

FIGS. 1A and 1B are a top view and a side sectional view, respectively, of an insertion device for an intraocular lens that is an embodiment of the present invention.

In the description below, a direction of pushing out an intraocular lens is referred to as a front, a front end direction or a front end side, and an opposite side is referred to as a rear, a rear end direction or a rear end side. An axis extending between the front end and the rear end side and passing through the center of the intraocular lens held in the insertion device is referred to as a central axis of the insertion device, and a direction along the central axis is referred to as an axial direction. Further, a direction perpendicular to the axial direction is referred to as a radial direction, and a direction around the axis (a direction along an outer periphery or an inner periphery in axial view) is referred to as a circumferential direction.

In FIGS. 1A and 1B, reference numeral 2 denotes a cylindrical member, and reference numeral 5 denotes an insertion cylinder mounted to a front end of the cylindrical member 2. The cylindrical member 2 and the insertion cylinder 5 constitute a main body.

A flange portion 2e extending radially outward is formed on a rear outer periphery of the cylindrical member 2. The flange portion 2e is a portion on which an operator places his/her finger when inserting a pushing shaft 6 described below in the front end direction.

In a rear upper surface of the insertion cylinder 5, an opening is formed for placing a lens support member (a first holding portion) 4 in the insertion cylinder 5, the lens support member 4 being used for supporting the intraocular lens (hereinafter simply referred to as a lens) 1 in the insertion cylinder 5. The lens 1 placed in the lens support member 4 (the insertion cylinder 5) through the upper surface opening is held by the lens support member 4 and a lens retaining portion (a second holding portion) formed in a cover member 3 that covers the upper surface opening so that a marginal (peripheral) portion around an optical portion (that is, a portion having an optical function as a lens) of the lens 1 is vertically held. The lens support member 4 and the lens retaining portion of the cover member 3 constitute a lens holding portion. A detailed configuration of the lens holding portion will be described later.

The lens 1 is held in a state in which a stress is not substantially applied to the optical portion by the first lens holding portion and the second lens holding portion. The state in which a stress is not substantially applied denotes a state in which no stress is applied to the optical portion at all as well as a state in which a minute stress is applied so that a deformation influencing the optical function of the optical portion after insertion of the lens 1 into an eye do not occur even if the lens 1 is held and stored for a long time.

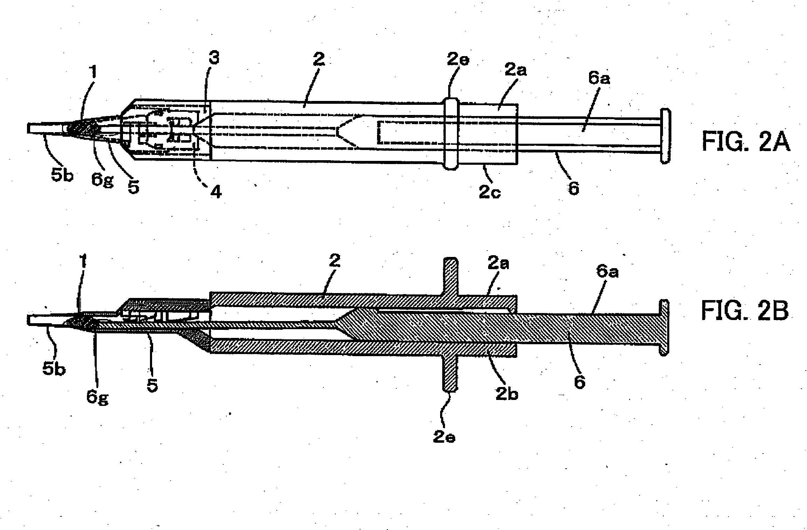

A front end 5b of the insertion cylinder 5 is inserted into an incision formed in an eyeball and feeds the lens 1 into the eye, and has a decreasing inner diameter toward the tip. As shown in FIGS. 2A and 2B (FIG. 2A is a top view and FIG. 2B is a side sectional view), the front end 5b folds and deforms the lens 1 into a small shape which is pushed by the pushing shaft 6 and passes through the inside of the front end 5b, and feeds the lens 1 from a front end opening into the eye.

In a portion closer to the front end than the axial middle of the pushing shaft 6 inserted into the cylindrical member 2, a pushing shaft portion 6e is formed having a small diameter that can pass through the front end 5b of the insertion cylinder 5. At the front end of the pushing shaft portion 6e, a bifurcated lens catching portion 6g is formed that vertically holds the optical portion of the lens 1 held by the lens holding portion and reliably pushes the lens 1 into the eye through the insertion cylinder 5.

In the middle of the pushing shaft 6, a cylindrical portion 6f is formed having a circular section in axial view of the pushing shaft 6 and having an outer diameter slightly smaller than an inner diameter of the cylindrical member 2. An unshown O-ring is mounted to an outer periphery of the cylindrical portion 6f, and brought into tight contact with an inner peripheral surface of the cylindrical member 2 to form a seal structure. A viscoelastic material such as sodium hyaluronate solution is introduced into the insertion cylinder 5 and the cylindrical member 2 as a lubricant when the lens is pushed into the eye, and the seal structure is provided for preventing the solution from leaking from a rear end opening of the cylindrical member 2.

A portion of the pushing shaft 6 closer to the rear end than the cylindrical portion 6f is formed to have a D-shaped section with a flat upper surface in axial view of the pushing shaft 6. Reference numeral 6a in FIG. 1 denotes a D-cut shaft portion having the D-shaped section. Further, a flange portion 6c extending radially outward beyond the D-cut shaft portion 6a and the cylindrical portion 6f is formed at the rear end of the pushing shaft 6. The flange portion 6c is pushed by the operator when pushing the pushing shaft 6 in the front end direction for inserting the lens 1 into the eye.

A portion closer to the rear than the flange portion 2e of the cylindrical member 2 has a divided structure circumferentially openable and closable.

The divided structure portion includes an openable and closable portion 2a and a fixed portion 2b. The fixed portion 2b is a semicylindrical portion formed to directly extend, rearward from the portion of the cylindrical member 2 closer to the rear end than the flange portion 2e. One end in the circumferential direction of the openable and closable portion 2a is connected to one end in the circumferential direction of the fixed portion 2b via a hinge portion 2c. The openable and closable portion 2a and the hinge portion 2c are formed integrally with the fixed portion 2b (that is, the cylindrical member 2). The openable and closable portion 2a is openable and closable with respect to the fixed portion 2b around the hinge portion 2c.

Though not shown, a lock pawl is formed on the side opposite to the hinge portion 2c of the openable and closable portion 2a. A lock receiving portion engageable with the lock pawl is formed on the side opposite to the hinge portion 2c of the fixed portion 2b. The openable and closable portion 2a is closed with respect to the fixed portion 2b to cause the lock pawl to engage the lock receiving portion, and thus the openable and closable portion 2a is locked in a state in which it is assembled (connected) to the fixed portion 2b.

A wall portion 2d is formed in the circumferential middle at the rear end of the openable and closable portion 2a. The wall portion 2d is provided for forming a rear end opening having a D-shaped section in the divided structure portion in a state in which the openable and closable portion 2a is closed with respect to the fixed portion 2b.

The shape of the rear end opening substantially matches the D-shaped section of the D-cut shaft portion 6 of the pushing shaft 6 to prevent rotation of the pushing shaft 6 with respect to the cylindrical member 2. The lens catching portion 6g provided at the front end of the pushing shaft 6 is oriented so as to properly catch upper and lower surfaces of the optical portion of the lens 1. Thus, as described above, preventing rotation of the pushing shaft 6 can prevent a pushing error of the lens 1.

In a boundary between the cylindrical portion 6f and the D-cut shaft portion 6a of the pushing shaft 6, a step 6b is formed by a difference in radial dimension between the D-cut shaft portion 6a and the cylindrical portion 6f. The step 6b contacts the wall portion 2d of the openable and closable portion 2a from the front end side to prevent removal of the pushing shaft 6 rearward from the cylindrical member 2. The removal preventing structure is provided for avoiding inconvenience of the removal of the pushing shaft 6 from the cylindrical member 2 during an operation.

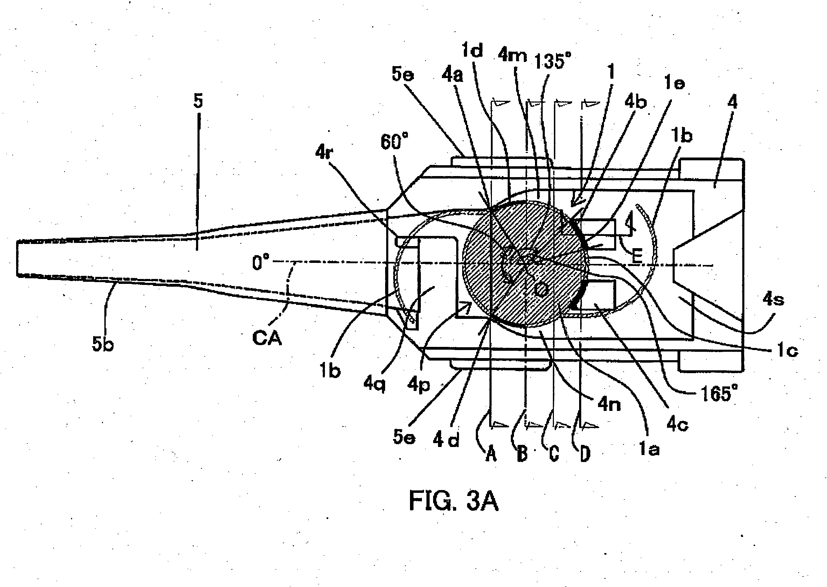

Next, the configuration of the lens holding portion will be described with reference to FIGS. 3A to 6.

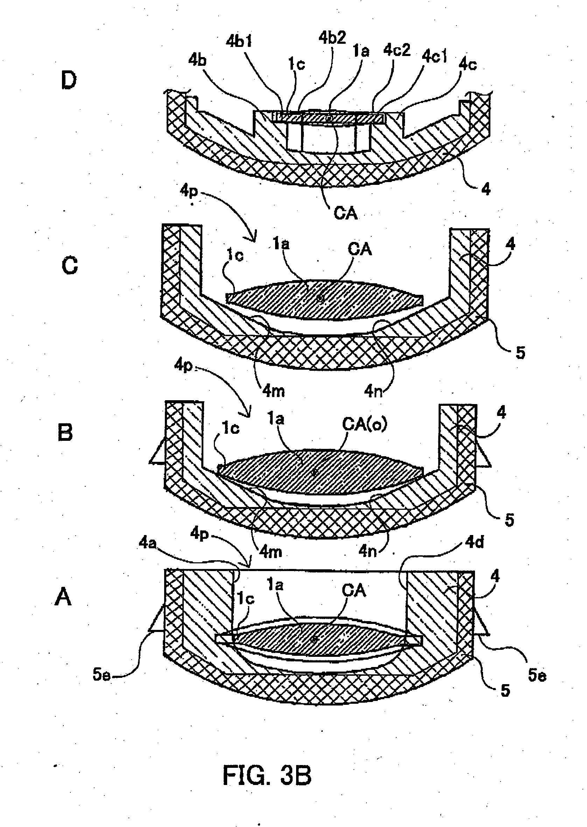

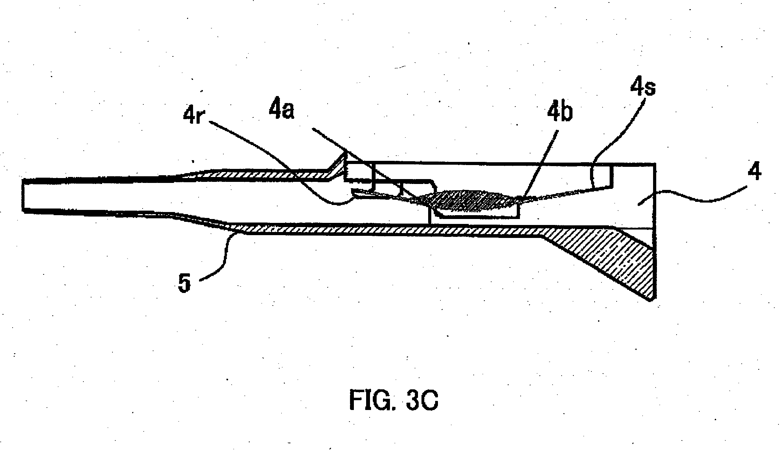

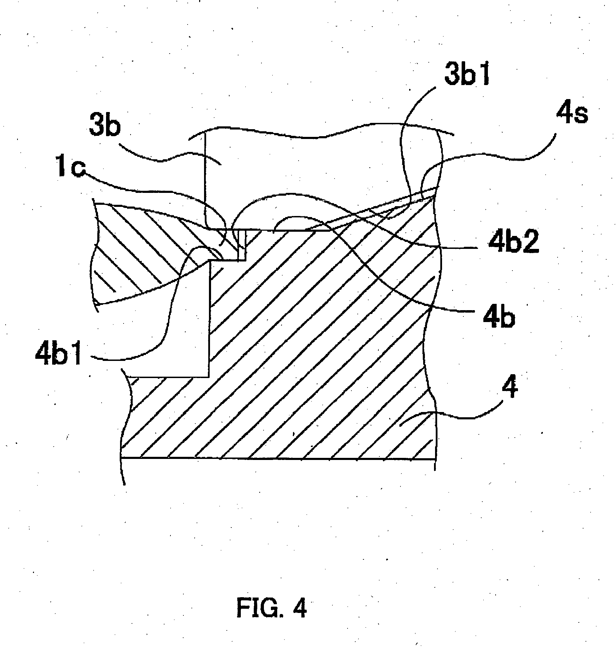

FIG. 3A shows the lens support member 4 assembled into the insertion cylinder 5 viewed from above (in the direction of a lens optical axis passing through the center O of the lens). FIG. 3B shows front sections of the lens support member 4 and the insertion cylinder 5 in FIG. 3A taken in the direction of arrows (from the front end side) at positions A to D in FIG. 3A. FIG. 3C shows a side section of the lens support member 4 and the insertion cylinder 5. FIG. 4 shows a section of part of the lens support member 4 taken in the direction of arrow at a position E in FIG. 3A.

In the description below, a surface (first surface) of the lens 1 supported by the lens support member 4 is referred to as a lower surface, and a surface (second surface) retained by a lens retaining portion of the cover member 3 described later is referred to as an upper surface. A vertical direction in FIG. 3B is a vertical direction of the lens support member 4, and a lateral direction in FIG. 3B is a lateral (horizontal) direction of the lens support member 4.

First, the configuration of the lens 1 held by the lens holding portion will be described. The lens 1 has a circular shape in top view, and includes an optical portion 1a having the function of a lens and support portions 1b extending from the front end and the rear end of the optical portion 1a.

The support portion 1b is a wire-like portion that elastically supports the optical portion 1a in the eye after the lens 1 is inserted into the eye.

A ring-shaped marginal portion 1c having upper and lower surfaces parallel to each other is formed around the optical portion 1a.

As shown in FIG. 3A, the lens support member 4 is formed symmetrically with respect to the central axis CA in top view except part thereof. Also, as shown in FIG. 3B, the lens support member 4 is formed symmetrically with respect to a surface (not shown) including the central axis CA and vertically extending in front view except part thereof.

The lens support member 4 is assembled into the insertion cylinder 5 through the upper surface opening in the insertion cylinder 5. An opening 4p through which the lens 1 is inserted is formed in the upper portion of the lens support member 4.

Support surfaces 4m and 4n as front side holding portions are formed on the right and left in the lower portion of the lens support member 4. The support surfaces 4m and 4n are formed as inclined surfaces whose inner portion is lower than its outer portion in the lateral direction. As shown in FIG. 3A, the support surfaces 4m and 4n contact the lower surfaces of arcuate regions (first regions) 1d from positions corresponding to the section A to positions corresponding to the section B in the marginal portion (hereinafter referred to as the lens marginal portion) 1c of the lens 1, and support the arcuate regions 1d from below.

As shown in FIG. 3A, the section A passes through a positions retracted by a first circumferential angle of 60° to both circumferential sides (hereinafter referred to as 60° positions) from a 0° position in the lens marginal portion 1c, the 0° position being a position in the front end direction from the center 0 of the lens 1 in the lens marginal portion 1c. The section B passes through positions retracted by a second circumferential angle of 90° to both circumferential sides (hereinafter referred to as 90° positions, and other positions are hereinafter referred to as the same) from the 0° position in the lens marginal portion 1c. Specifically, the support surfaces 4m and 4n support the arcuate regions 1d from the 60° positions to the 90° positions in the lens marginal portion 1c.

The section C passes through 120° positions retracted by a circumferential angle of 120° to both circumferential sides from the 0° position in the lens marginal portion 1c. Further, the section D passes through 150° positions retracted by a circumferential angle of 150° to both circumferential sides from the 0° position in the lens marginal portion 1c. A position opposite to the 0° position with respect to the lens center O is a 180° position.

On the right and left of the lens support member 4, support protrusions 4b and 4c are formed as rear side holding portions that support arcuate regions (second regions) 1e from 135° positions to 165° positions which are regions closer to the rear than the arcuate regions 1d supported by the support surfaces 4m and 4n. A space through which the pushing shaft 6 passes is provided between the support protrusions 4b and 4c. Since the space has only a 30° angle range, it may be considered that the support protrusions 4b and 4c support an arcuate region of a 90° angle range around the 180° position in the lens marginal portion 1c.

Specifically, the lens support member 4 of the embodiment can be said that it supports the optical portion 1a of the lens 1 at three points at 120° intervals including the right and left 60° positions and the 180° position in the lens marginal portion 1c.

As shown in an enlarged manner in FIG. 4, the support protrusion 4b has a horizontal surface 4b1 and a vertical surface 4b2 formed on the rear side of the horizontal surface 4b1. The horizontal surface 4b1 has an arcuate shape in top view, and contacts a lower surface of the arcuate region 1e to support the arcuate region 1e from below. The vertical surface 4b2 is an arcuate surface that contacts or is brought close to an outer peripheral end surface of the arcuate region 1e, and prevents rearward movement of the lens 1. As shown in a D-sectional view in FIG. 3B, the support protrusion 4c also has a horizontal surface 4c1 and a vertical surface 4c2.

As shown in the A sectional view in FIG. 3B, on the right and left on the front end side of the lens support member 4, vertical surfaces 4a and 4d as third holding portions are formed that contact or are brought close to the 60° positions in the outer peripheral end surface of the lens marginal portion 1c, that is, the positions in the direction of the first circumferential angle. The vertical surfaces 4a and 4d prevent movement of the lens in the front end direction in a state before pushing out the lens 1.

As shown in FIG. 3A, in an upper portion at the front end of the lens support member 4, an arm 4q is formed extending from the right to the left in FIG. 3B (from the lower side to the upper side in FIG. 3A), and at the left end of the arm 4q, a protrusion 4r that supports from below the front side support portion 1b of the lens 1 is formed to extend in the front end direction.

On the rear of the lens support member 4, an inclined surface 4s that supports from below the rear side support portion 1b of the lens 1 is formed with a portion closer to the rear end being located in a higher position (see FIG. 3C).

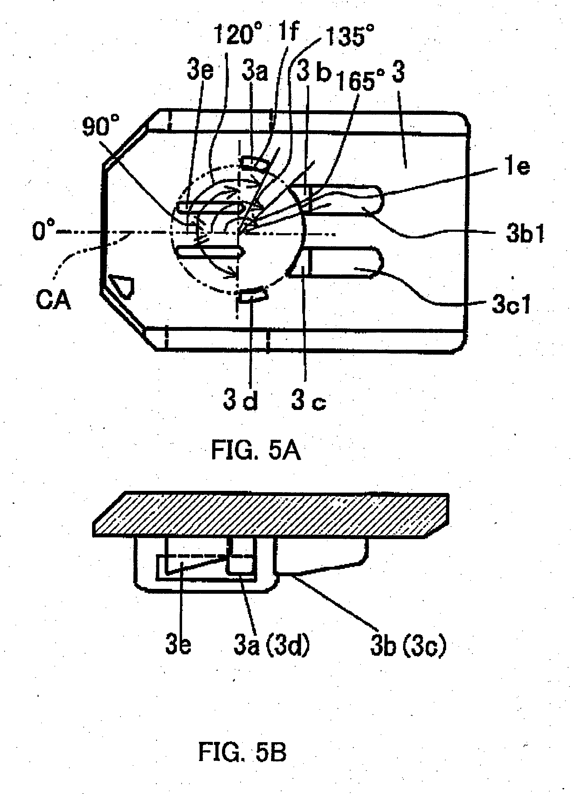

Next, the configuration of the lens retaining portion in the cover member 3 will be described with reference to FIGS. 5A and 5B (FIG. 5A is a bottom view and FIG. 5B is a side sectional view). A vertical direction in FIG. 5B is a vertical direction of the lens support member 4, and a vertical direction in FIG. 5A is a lateral (horizontal) direction of the lens support member 4.

The cover member 3 is placed to cover the upper surface opening 4p in the lens support member 4, and locked by lock pawls 5e formed on the side surface of the insertion cylinder 5.

The cover member 3 is formed symmetrically with respect to the central axis CA in top view. The cover member 3 is also formed symmetrically with respect to a surface (not shown) including the central axis CA and vertically extending in front view.

On the right and left in the lower surface of the cover member 3, retaining protrusions 3a and 3d are formed as front side holding portions that contact or are brought close to arcuate regions (third regions) 1f from the 90° positions to substantially the 120° positions, the 90° positions being retracted by a circumferential angle of 90° to both circumferential sides from the 0° position in the upper surface of the lens marginal portion 1c. Retaining protrusions 3b and 3c are also formed as rear side holding portions that contact or are brought close to arcuate regions 1g from the 135° positions to the 165° positions, the 135° positions being retracted by a circumferential angle of 135° to both circumferential sides from the 0° position in the upper surface of the lens marginal portion 1c. A space through which the pushing shaft 6 passes is provided between the retaining protrusions 3b and 3c.

The retaining protrusions 3b and 3c are placed to retain from above the arcuate regions 1e in the lens marginal portion 1c supported from below by the support protrusions 4b and 4c provided in the lens support member 4. On the other hand, the retaining protrusions 3a and 3d are placed to retain from above the arcuate regions 1f (the regions closer to the rear than the arcuate regions 1d that contact the support surfaces 4m and 4n) in the lens marginal portion 1c that does not contact the support surfaces 4m and 4n provided in the lens support member 4.

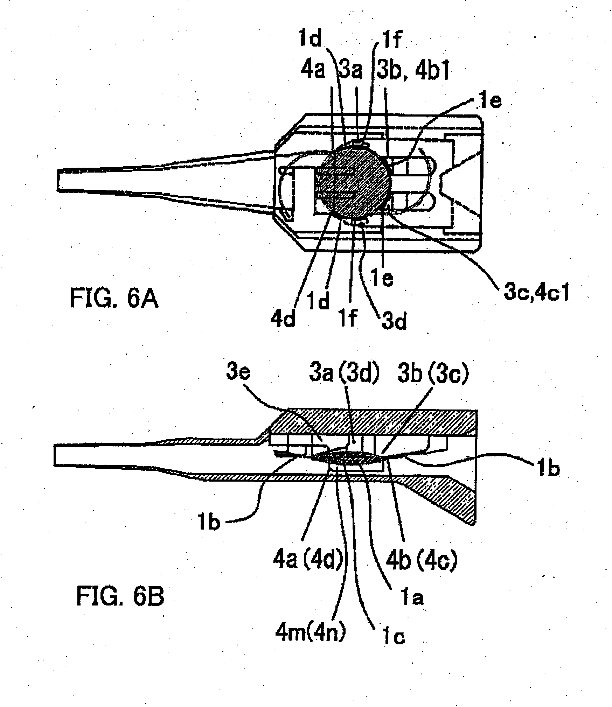

In the lens holding portion configured as described above, as shown in FIGS. 6A and 6B (FIG. 6A is a top view and FIG. 6B is a side sectional view) showing a state in which the cover member 3 is assembled to the lens support member 4, the arcuate regions 1d from the 60° positions to the 90° positions in the lens marginal portion 1c are supported by the support surfaces 4m and 4n of the lens support member 4, and the arcuate regions 1f from the 90° positions to the 120° positions are retained from above by the retaining protrusions 3a and 3d of the cover member 3. The arcuate regions 1e from the 135° positions to the 165° positions are vertically held by the horizontal surfaces 4b1 and 4c1 of the support protrusions 4b and 4c provided in the lens support member 4 and the retaining protrusions 3b and 3c of the cover member 3 therebetween. With such a holding structure, the lens 1 is supported in a state in which the optical portion 1a of the lens 1 is held in a horizontal state and a stress by its own weight or an external force is not substantially applied.

Further, the vertical surfaces 4a, 4d, 4b2 and 4c2 that contact the 60° positions and the regions from the 135° positions to the 165° positions in the outer peripheral end surface of the lens 1 prevent a shift of the lens 1 in the front end direction and the rear end direction.

In portions of the retaining protrusions 3b and 3c in the cover member 3 closer to the rear than the portions retaining the lens marginal portion 1c, inclined portions 3b1 and 3c1 are formed that extend in parallel with the inclined surface 4s of the lens support member 4 and hold the rear side support portion 1b of the lens 1 together with the inclined surface 4s.

Further, the vertical surface 4a is formed to extend in the front end direction along an outer edge of the front side support portion 1b of the lens 1. The contact of the vertical surface 4a with the front side support portion 1b and the holding of the rear side support portion 1b between the inclined surface 4s and the inclined portions 3b1 and 3c1 prevent rotation of the lens 1. The vertical surface 4d has the same shape as that of the vertical surface 4a.

On the right and left of the cover member 3 on the front end side from the center O of the lens 1, deformation guide portions 3e are formed which are brought close to the upper surface (lens surface) of the optical portion 1a of the lens 1 held by the lens holding portion. The lower surface of the deformation guide portion 3e has a lower end surface that is an inclined surface with its front side lower than its rear side. The deformation guide portions 3e contact the upper surface of the optical portion 1a when the lens 1 is pushed by the pushing shaft 6 from the lens holding portion in the front end direction (before the lens 1 is completely separated from the lens holding portion), and function as a guide that deforms the optical portion 1a into a downward protruding shape. Thus, the lens 1 can be smoothly deformed into a small shape by the front end 5b of the insertion cylinder 5 after the lens 1 is completely separated from the lens holding portion.

Next, the relationship between the lens holding portion and the behavior of the lens 1 in pushing in the embodiment will be described.

In the lens holding portion of the embodiment, the lens 1 (the optical portion 1a) is horizontally held in a state in which the region having a 120° width around the central axis CA (the 0° position) in the lens marginal portion 1c is opened in the front end direction. Further, the arcuate regions 1f retained from above in the lens marginal portion 1c are shifted rearward with respect to the arcuate regions 1d supported from below. Thus, when a force in the front end direction is applied to the lens 1 by the pushing shaft 6, the lens 1 can be moved from the lens holding portion in the front end direction smoothly without high resistance.

The lens 1 is moved from the lens holding portion in the front end direction while being deformed into a downward protruding shape by the deformation guide portions 3e provided in the cover member 3. At this time, as described above, the arcuate regions 1f retained from above in the lens marginal portion 1c are shifted rearward with respect to the arcuate regions 1d (the regions contacting with the support surfaces 4m and 4n) supported from below, which does not prevent upward displacement of the arcuate regions 1d along with the deformation. This allows smooth deformation of the lens 1 that is moved from the lens holding portion in the front end direction.

As described above, according to the embodiment, the lens 1 can be held in a position in which a stress is not substantially applied to the optical portion 1a and the lens 1 can be directly pushed out by the pushing shaft 6 in the front end direction. Thus, a convenient insertion device is achieved that can store the lens 1 and can insert the lens into the eye simply by pushing the pushing shaft 6.

In an example not part of the invention, the plurality of circumferentially separated surfaces or protrusions of the lens support member 4 and the cover member 3 can contact the regions between the 0° position and the 180° position in the lens marginal portion 1c. This achieves a holding structure that allows horizontal holding of the optical portion 1a, and smooth pushing and deformation of the lens 1.

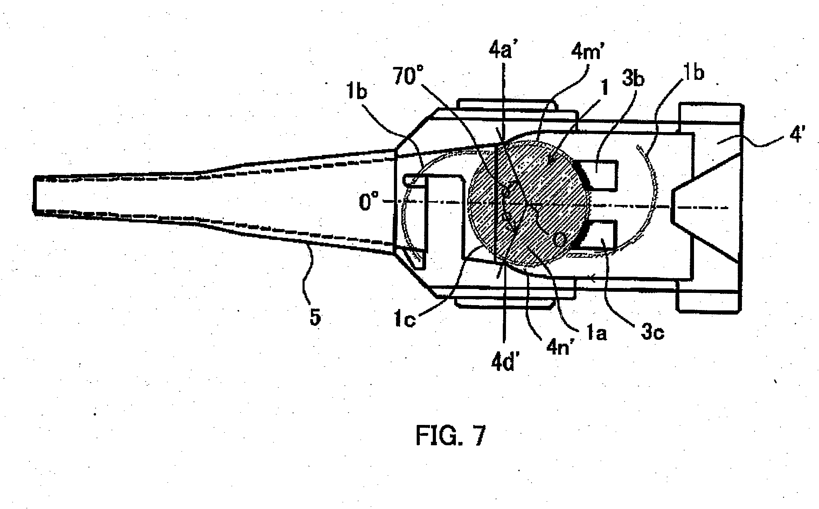

FIGS. 7 to 9 show the configuration of a lens holding portion of experimental comparative examples as compared with the lens holding portion of the insertion device of the embodiment. In FIGS. 7 to 9, the same components as in the embodiment are denoted by the same reference numerals.

### (Comparative Example 1)

FIG. 7 shows the configuration of a lens holding portion of Comparative Example 1. In the above embodiment, the arcuate regions 1d from the 60° positions to the 90° positions in the lens marginal portion 1c are supported by the support surfaces 4m and 4n, and the vertical surfaces 4a and 4d are formed that contact or are brought close to the 60° positions in the outer peripheral end surface of the lens marginal portion 1c. On the other hand, in Comparative Example 1, support surfaces 4m' and 4n' are formed to support arcuate regions from 70° positions to 90° positions in the lens marginal portion 1c, and vertical surfaces 4a' and 4d' are formed to contact or be brought close to the 70° positions in an outer peripheral end surface of the lens marginal portion 1c.

In this case, an opening angle (140°) of an optical portion 1a in the front end direction is larger than that in the embodiment (120°), thereby facilitating pushing out the lens 1 in the front end direction. However, as compared with the embodiment, the supported region by the support surfaces 4m' and 4n' in a lower surface of the lens marginal portion 1c are retracted rearward, and thus the front end side of the optical portion 1a is vertically displaced to prevent the lens holding portion from performing the function of stably holding the lens 1.

### (Comparative Example 2)

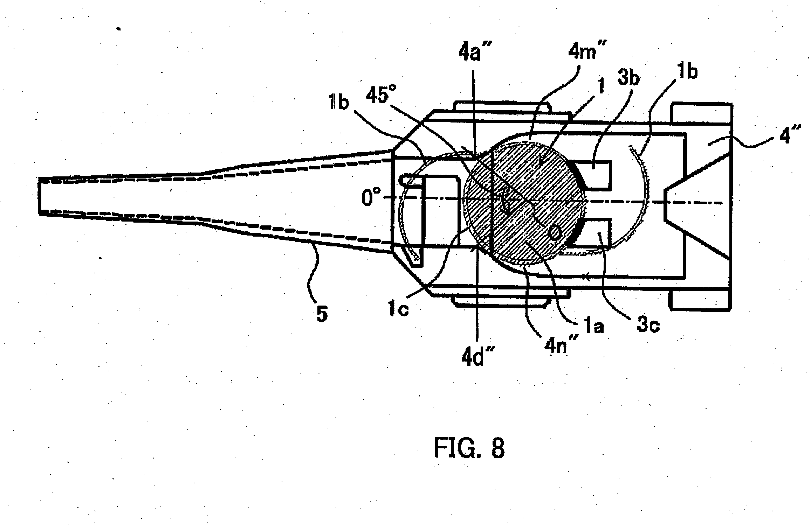

FIG. 8 shows the configuration of a lens holding portion of Comparative Example 2.

In Comparative Example 2, support surfaces 4m" and 4n" are formed to support arcuate regions from 45° positions to 90° positions in the lens marginal portion 1c, and vertical surfaces 4a" and 4d" are formed to contact or be brought close to the 45° positions in an outer peripheral end surface of the lens marginal portion 1c.

In this case, the lens 1 can be stably held, but an opening angle (90°) of the optical portion 1a in the front end direction is smaller than that in the embodiment (120°), which increases resistance when pushing the lens 1 in the front end direction to prevent the lens holding portion from performing the function of smoothly feeding the lens 1.

### (Comparative Example 3)

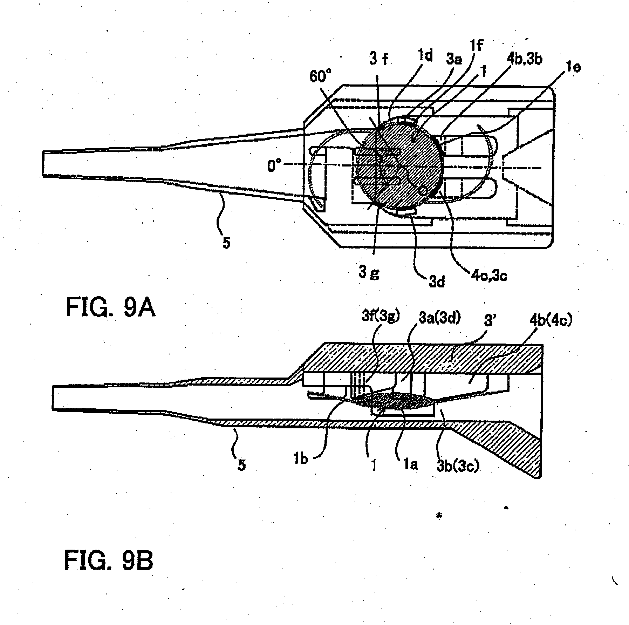

FIGS. 9A and 9B (FIG. 9A is a top view and FIG. 9B is a side sectional view) show the configuration of a lens holding portion of Comparative Example 3.

In the embodiment, the support surfaces 4m and 4n support the arcuate regions 1d from 60° positions to 90° positions in the lens marginal portion 1c, and the retaining protrusions 3a and 3d retain the arcuate regions 1f closer to the rear than the arcuate regions 1d (the rear from the 90° positions). On the other hand, in the Comparative Example 3, arcuate regions from the 60° positions to 70° positions in arcuate regions 1d are retained by retaining protrusions 3f and 3g that are not provided in the embodiment.

In this case, the lens 1 can be stably held, but the arcuate regions 1d cannot be displaced upward when the optical portion 1a is deformed into a downward protruding shape by the deformation guide portions 3e. This prevents smooth pushing and deformation of the lens 1.

A consideration based on the comparative examples has found that the support surfaces 4m and 4n most preferably support the regions rearward from the 60° positions in the lens marginal portion 1c as in the embodiment. However, alternative embodiments of the present Invention are not limited thereto. The support surfaces 4m and 4n support regions rearward from positions having a circumferential angle of larger than 45° and smaller than 70° with respect to the 0° position in the lens marginal portion 1c. Preferably, the support surfaces 4m and 4n may support regions rearward from positions having a circumferential angle from 55° or more to 65° or less with respect to the 0° position.

On the other hand, the retaining protrusions 3a and 3d most preferably retain the regions rearward from the 90° positions in the lens marginal portion 1c as in the embodiment. However, alternative embodiments of the present invention are not limited thereto. The retaining protrusions 3a and 3d retain regions rearward from positions having a circumferential angle of larger than 80° with respect to the 0° position. Preferably, the retaining protrusions 3a and 3d may retain regions rearward from positions having a circumferential angle of larger than 85° with respect to the 0° position.

As described above, according to the embodiment, for example, in the state in which the lens is placed in the lens holding portion with the first surface downward, the first holding portion supports the lens marginal portion in the regions rearward from positions closer to the front than the second holding portion (that is, the positions closer to the rear than the 45° positions and closer to the front than the 70° positions). This allows the lens to be supported without downward deformation of the front end side of the lens. Further, a sufficient opening angle range in the front end direction can be formed on the front end side of the lens, thereby allowing the lens held by the lens holding portion to be smoothly pushed out by the pushing shaft.

Further, the second holding portion retains the region closer to the rear than the first holding portion in the second surface of the lens marginal portion of the lens supported by the first holding portion. This reliably prevents the lens from being raised from the first holding portion, and allows pushing of the lens by the pushing shaft and deformation of the lens along therewith to be performed more smoothly than the case where the second holding portion contacts the same region as the first holding portion in the lens marginal portion.

The lens holding portion holds the lens marginal portion, thereby allowing the lens to be held in a state in which a stress is not substantially applied to the optical portion, and allowing the lens to be stored for a long time.

Furthermore, the present invention is not limited to the above preferred embodiments and various variations and modifications may be made within the scope of the appended claims.

For example, in the embodiment, the case is described where the lens support member 4 produced separately from the insertion cylinder 5 is assembled into the insertion cylinder 5, but in an alternative example of the present invention, the lens support member may be formed integrally with the insertion cylinder in structure or shape.

In the above embodiments, the insertion device for an intraocular lens having a wire-like support portion extending from the optical portion has been described, but as an alternative example of the present invention, an insertion device for an intraocular lens having a plate-like support portion extending around an optical portion is included. In this case, a lens marginal portion may include the plate-like support portion.

Furthermore, the present invention is not limited to these examples and various variations and modifications may be made within the scope of the appended claims.

An insertion device for an intraocular lens is disclosed in the appended claims which capable of pushing out the lens into an eye by a pushing shaft directly from a state in which the lens is stored. The device includes a lens holding portion (3, 4), and a pushing shaft (6) moving the lens (1) from the lens holding portion. The lens holding portion includes first and second holding portions allowed to contact surfaces opposite to each other of a marginal portion (1e) of the lens. The first holding portion (4) is allowed to contact regions (1d) rearward from positions having a first circumferential angle of larger than 45° and smaller than 70° to both circumferential sides from a 0° position in the marginal portion. The second holding portion (3) is allowed to contact regions (1f) rearward from positions having a second circumferential angle larger than the first circumferential angle in the marginal portion.

## Claims

1. An insertion device for an intraocular lens (1) comprising:
a lens holding portion (3, 4) that holds the lens; and
a pushing shaft (6) that moves the lens from the lens holding portion in a front end direction of the insertion device, the lens moved by the pushing shaft being inserted into an eye,
wherein the lens holding portion includes a first holding portion (4) which is allowed to contact a surface of a marginal portion (1c) of the lens on a first side of the lens and a second holding portion (3) which is allowed to contact another surface of the marginal portion of the lens on a second side of the lens opposite to the first side,
when a position in the front end direction in the marginal portion with respect to the center of the lens is a front end position, wherein
the second holding portion (3) includes a deformation guide portion (3e) that is adapted to deform the lens into a shape convexly protruding toward the first side when the pushing shaft (6) moves the lens from the lens holding portion (3, 4) in the front direction,
**characterized in that**
the first holding portion (4) is allowed to contact regions (1d) rearward from positions having a first circumferential angle larger than 45° and smaller than 70° to both circumferential sides from the front end position in the marginal portion, and
the second holding portion (3) is allowed to contact regions (1f) rearward from positions having a second circumferential angle larger than 80° to both circumferential sides from the front end position in the marginal portion.

2. An insertion system for an intraocular lens (1) comprising:
the insertion device according to claim 1; and
the intraocular lens (1) held by the lens holding portion (3, 4).

3. A method for manufacturing an insertion system for an intraocular lens (1) comprising the steps of:
preparing the insertion device according to claim 1; and
placing the intraocular lens in the lens holding portion (3, 4).

## Patentansprüche

1. Einsetzvorrichtung für eine intraokulare Linse (1) mit:
einem Linsenhalteabschnitt (3, 4), der die Linse hält; und
einem Schiebeschaft (6), der die Linse von dem Linsenhalteabschnitt in einer Vorderendrichtung der Einsetzvorrichtung bewegt, wobei die durch den Schiebeschaft bewegte Linse in ein Auge eingesetzt wird,
wobei der Linsenhalteabschnitt einen ersten Linsenhalteabschnitt (4), dem erlaubt wird, mit einer Fläche eines Randabschnitts (1c) der Linse an einer ersten Seite der Linse in Kontakt zu kommen, und einen zweiten Halteabschnitt (3) aufweist, dem erlaubt wird, mit einer anderen Fläche des Randabschnitts der Linse an einer zweiten Seite der Linse, die der ersten Seite entgegengesetzt ist, in Kontakt zu kommen,
wenn eine Position in der Vorderendrichtung des Randabschnitts mit Bezug auf die Mitte der Linse eine Vorderendposition ist, wobei
der zweite Halteabschnitt (3) einen Verformungsführungsabschnitt (3e) aufweist, der dazu angepasst ist, die Linse in eine Form zu verformen, die zu der ersten Seite konvex vorragt, wenn der Schiebeschaft (6) die Linse von dem Linsenhalteabschnitt (3, 4) in der Vorderendrichtung bewegt,
**dadurch gekennzeichnet, dass**
dem ersten Halteabschnitt (4) erlaubt wird, mit Bereichen (1d) in Kontakt zu kommen, die hinter Positionen liegen, die einen ersten Umfangswinkel, der größer als 45° und kleiner als 70° ist, zu beiden Umfangsseiten von der Vorderendposition in dem Randabschnitt haben, und
dem zweiten Halteabschnitt (3) erlaubt wird, mit Bereichen (1f) in Kontakt zu kommen, die hinter Positionen liegen, die einen zweiten Umfangswinkel, der größer als 80° ist, zu beiden Umfangsseiten von der Vorderendposition in dem Randabschnitt haben.

2. Einsetzsystem für eine intraokulare Linse (1) mit:
der Einsetzvorrichtung gemäß Anspruch 1; und
der intraokularen Linse (1), die durch den Linsenhalteabschnitt (3, 4) gehalten ist.

3. Verfahren zum Herstellen eines Einsetzsystems für eine intraokulare Linse (1) mit den Schritten:
Bereitstellen der Einsetzvorrichtung gemäß Anspruch 1; und
Platzieren der intraokularen Linse in dem Linsenhalteabschnitt (3, 4).

## Revendications

1. Dispositif d'insertion pour une lentille intraoculaire (1) comprenant :
une partie de maintien de lentille (3, 4) qui maintient la lentille ; et
un arbre de poussée (6) qui déplace la lentille depuis la partie de maintien de lentille dans une direction de l'extrémité avant, la lentille déplacée par l'arbre de poussée étant insérée dans un oeil,
dans lequel la partie de maintien de lentille inclut une première partie de maintien (4) qui est laissée entrer en contact avec une surface d'une partie marginale (1c) de la lentille sur un premier côté de la lentille et une deuxième partie de maintien (3) qui est laissée entrer en contact avec une autre surface de la partie marginale de la lentille sur un deuxième côté de la lentille opposé au premier côté,
quand une position dans la direction de l'extrémité avant dans la partie marginale par rapport au centre de la lentille est une position d'extrémité avant, dans lequel
la deuxième partie de maintien (3) inclut une partie de guide de déformation (3e) qui est adaptée pour déformer la lentille en une forme saillant de manière convexe vers le premier côté quand l'arbre de poussée (6) déplace la lentille depuis la partie de maintien de lentille (3, 4) dans la direction avant,
**caractérisé en ce que**
la première partie de maintien (4) est laissée entrer en contact avec des régions (1d) vers l'arrière de positions ayant un premier angle circonférentiel supérieur à 45° et inférieur à 70° aux deux côtés circonférentiels de la position de l'extrémité avant dans la partie marginale, et
la deuxième partie de maintien (3) est laissée entrer en contact avec des régions (1f) vers l'arrière de positions ayant un deuxième angle circonférentiel supérieur à 80° aux deux côtés circonférentiels de la position de l'extrémité avant dans la partie marginale.

2. Système d'insertion pour une lentille intraoculaire (1) comprenant :
le dispositif d'insertion selon la revendication 1 ; et
la lentille intraoculaire (1) maintenue par la partie de maintien de lentille (3, 4).

3. Procédé de fabrication d'un système d'insertion pour une lentille intraoculaire (1) comprenant les étapes de :
préparer le dispositif d'insertion selon la revendication 1 ; et
placer la lentille intraoculaire dans la partie de maintien de lentille (3, 4).
